(19) Europäisches Patentamt
European Patent Office
Office européen des brevets

(11) EP 1 391 193 B1

(12) EUROPÄISCHE PATENTSCHRIFT

(45) Veröffentlichungstag und Bekanntmachung des Hinweises auf die Patenterteilung:
**03.01.2007 Patentblatt 2007/01**

(51) Int Cl.:
***A61K 8/894*** *(2006.01)* ***A61K 8/06*** *(2006.01)*
***A61Q 17/04*** *(2006.01)*

(21) Anmeldenummer: **03013596.6**

(22) Anmeldetag: **14.06.2003**

(54) **Silikon-in-Wasser-Emulsion enthaltend spezifisches Polyether-Polydimethylsiloxan-Copolymer**

Silicone-in-water emulsion comprising specific polyether-polydimethylsiloxane copolymer

Emulsion silicone-dans-eau comprenant un copolymère polyether-polydimethylsiloxane specifique

(84) Benannte Vertragsstaaten:
**AT BE BG CH CY CZ DE DK EE ES FI FR GB GR HU IE IT LI LU MC NL PT RO SE SI SK TR**

(30) Priorität: **31.07.2002 DE 10234885**

(43) Veröffentlichungstag der Anmeldung:
**25.02.2004 Patentblatt 2004/09**

(73) Patentinhaber: **Beiersdorf AG**
**20245 Hamburg (DE)**

(72) Erfinder:
- **Göppel, Anja**
  **22527 Hamburg (DE)**
- **Bürger, Anette**
  **22303 Hamburg (DE)**
- **Pfannenbecker, Uwe**
  **22419 Hamburg (DE)**
- **Schwanke, Frank, Dr.**
  **20257 Hamburg (DE)**
- **Raschke, Thomas, Dr.**
  **25421 Pinneberg (DE)**
- **Nielsen, Jens**
  **24558 Henstedt-Ulzburg (DE)**
- **Syskowski, Boris**
  **22303 Hamburg (DE)**

(56) Entgegenhaltungen:
**EP-A- 0 154 837**
**EP-A- 0 516 547**
**EP-A- 1 010 717**
**EP-A- 1 149 872**
**WO-A-03/064500**

- **DIETZ T: "A NOVEL SILICONE-BASED O/W EMULSIFIER WITH SKIN SMOOTHING SENSATION" COSMETICS & TOILETRIES, WHEATON, IL, US, Bd. 117, Nr. 5, Mai 2002 (2002-05), Seiten 71-74,76,78,80-81, XP009014858 ISSN: 0361-4387**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 391 193 B1

## Beschreibung

Die vorliegende Erfindung betrifft kosmetische und/oder dermatologische Zubereitungen in. Form von Silikon-in-Wasser Emulsionen, die bestimmte Silikonemulgatoren und in besonderen Ausführungsformen zusätzlich Tenside enthalten.

Die vorliegende Erfindung betrifft auch Sonnenschutzzubereitungen mit verringertem Klebrigkeitsgefühl, sowie deren Verwendung.

Die vorliegende Erfindung betrifft ferner wirkstoffhaltige kosmetische und/oder dermatologische Zubereitungen, in denen die Wirkstoffe auf besondere Weise stabilisiert sind.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W Emulsion, z. B. Milch). Der Grundcharakter einer O/W Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Gewöhnliche O/W-Emulsionen enthalten üblicherweise nur 5 bis 10 Gew.% Silikonöle, da sich höhere Mengen zumeist schlecht langzeitstabil einarbeiten lassen. Daher sind bisher nur W/Si-Emulsionen bekannt, haben jedoch wegen ihrer stark fettigen Sensorik in Kosmetik und Dermatologie nur eine geringe Verbreitung gefunden.

Emulgatoren auf der Basis von Silikonen an sich sind bereits bekannt.

Die Gesellschaft Goldschmidt AG bietet unter der Bezeichnung Abil Care 85 ® (INCI: Bis-PEG/PPG-16/16 PEG/PPG16/16 Dimethicone; Caprylic/Capric Triglyceride) einen silikonbasierten nichtionischen Emulgator für O/W-Emulsionen an, bei dem es sich um eine klare Flüssigkeit mit einem HLB-Wert von etwa 10 handelt. Chemisch ist es eine Mischung aus partiell alkoxylierten Polydimethylsiloxanen und einer Mischung der Ester aus Glycerin und Capryl- und/oder Caprinsäure, der keine unveresterten Hydroxylgruppen aufweist.
Dieser O/W-Emulgator zeichnet sich durch ein langanhaltendes, seidig-weiches Hautgefühl der unter seiner Verwendung hergestellten O/W-Emulsionen aus. Der Emulgator ist farb- und geruchlos und hat einen Erstarrungspunkt < 0°C.

Der Einsatz von Tensiden in kosmetischen und/oder dermatologischen Zubereitungen ist an sich bekannt. Tenside im Sinne der Erfindung umfassen die Gruppe der tensidischen Betaine, Alkylpolyglycoside, Aminosäurederivate sowie der quaternären Ammoniumverbindungen.

Fettsäurebetaine stellen Derivate des N,N,N-Trimethylglycins, auch als Betain bezeichnet dar, in denen mindestens eine Methylgruppe durch langkettige Alkylreste mit 10 bis 14 Kohlenstoffatomen ersetzt ist.

Fettalkohol- und Fettsäureglucoside sind Verbindungen aus Fettalkoholen oder Fettsäuren mit Glucose, wobei insbesondere Fettsäuren mit 8 bis 16 Kohlenstoffatomen und Fettalkohole mit 12 bis 16 Kohlenstoffatomen von Interesse sind.

Die Salze langkettiger Aminosäuren, die aus Amino- u. Fettsäuren hergestellt werden, sind Netzmittel, die nicht nur gut wasserlöslich sind, gut u. schonend waschen u. beständig sind gegen die Härtebildner des Wassers, sondern auch einen günstigen Einfluß auf den Haut- u. Haarzustand nehmen können. Es wird ihnen nachgesagt, daß sie als Antireizstoffe wirken.

Quaternäre Ammoniumverbindungen sind erhältlich durch Alkylierung teriärer Amine. Zu dieser Stoffgruppe gehören Alkylammonium-, Imidazolinium- und Pyridinium-Verbindungen. Sie werden als oberflächenaktive Stoffe oder auch als Mikrobizide verwendet.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem/Anlage 7 der Kosmetikverordnung zusammengefasst.

Um die Wirksamkeit der Lichtschutzfilter für die Haut abschätzen zu können, wurde in den 50er Jahren der Lichtschutzfaktor (LSF oder LF) bzw. Sonnenschutzfaktor (SF, engl. sun protection factor SPF) von Schulze eingeführt. Er definiert sich wie folgt:

$$LSF = \frac{MED\ geschützte\ Haut}{MED\ ungeschützte\ Haut}$$ MED = Erythemschwellendosis (engl. minimum erythemal dose)

Die europäische Patentanmeldung 627259 offenbart ein Verfahren zum Herstellen einer Emulsion, indem eine Ölphase zu einer Wasserphase gegeben und anschließend die Phasen miteinander gemischt werden, wobei die Ölphase Silikonöl und ein Silikonoxalkylen-Copolymer umfasst sowie die wäßrige Phase ein weiteres Silikonoxalkylen-Copolymer enthält und der HLB-Wert der Mischung der Silikonoxalkylen-Copolymere zwischen 4 und 7 liegt. Über einen Einsatz von Silikonoxalkylen-Copolymeren mit einem HLB-Wert zwischen 9 und 11 wird dagegen nichts offenbart.

Die deutsche Patentschrift 4241799 beschreibt eine aus zwei getrennt voneinander vorliegende Phasen bestehende kosmetische Zubereitung, wobei sich die beiden Phasen durch Schütteln bei der Anwendung zu einer Emulsion vereinigen, die sich anschließend wieder in die beiden Phasen trennt und die 0,05 bis 5 Gew.% eines bestimmten Silikon-Copolyols enthält. Demgegenüber wird über Zubereitungen, die lange Zeit als stabile Emulsionen vorliegen nichts offenbart.

Die europäische Patentschrift 516547 beschreibt stabile kosmetische O/W oder Si/W-Emulsionen, deren Ölphase im wesentlichen aus Silikonöl besteht und deren Emulgator ein bestimmtes Polyorganosiloxan-Polyethylen ist. Über den Einsatz von Silikonoxalkylen-Copolymeren, bei denen "-alkylen" nicht allein "-ethylen" bedeutet, wird nichts offenbart.

Die europäische Patentschrift 279319 beschreibt kosmetische O/W oder Si/W-Emulsionen, deren Ölphase bestimmte hydrophob beschichtete Pigmete sowie bestimmte Silikonöle, außerdem Wasser und bestimmte Polydiorganosiloxan-Polyalkylen-Copolymere enthält. Über Polydiorganosiloxan-Polyalkylen-Copolymere, die lange alkoxylierte Siloxanseitenketten aufweisen wird nichts offenbart.

Die europäische Patentschrift 154837 beschrteibt kosmetische Emulsionen, die 0,2 bis 5 Gew.% einer bestimmten Emulgatorzubereitung aus Dimethylpolysiloxan-polyoxyalkylen-Copolymer, einer oberflächenaktiven Substanz mit einem HLB-Wert größer oder gleich 10 und einem linearen C12-C22-Alkanol sowie 0,5 bis 20 Gew.% eines bestimmten Silikonöls und eine wäßrige, alkoholhaltige Phase enthalten. Über alkoholfreie Emulsionen wird dagegen nichts offenbart.

Die europäische Patentanmeldung 1125574 beschreibt O/W oder Si/W-Emulsionen, die ein oder mehrere Polyethersiloxane der allgemeinen Formel (I)

$$R\text{-}((CH_{32}SiO)_m\text{-}Si(CH_3)R\text{-}(O(CH_3)_2Si)_nR \qquad (I)$$

wobei n = 50 bis 250, $R = -(CH_2)_m-O-(C_2H_4O)_x-(C_3H_6O)_yR^1$, m = 2 bis 4, x = 3 bis 100, y = 0 bis 50 und $R^1$ = H, Methyl oder Ethyl ist, mit einem Gewichtsanteil der Polyetherreste R von bis zu 45 Gew.% an der Gesamtmolekularmasse, berechnet nach Formel (II)

$$\text{„Gewichtsanteil der Polyetherreste R an der Gesamtmolekularmasse“} = (MG_{Polyetherreste}/MG_{Gesamt}) \cdot 100 \qquad (II)$$

mit $MG_{Gesamt} = MG_{Silikonrest} + MG_{Polytherreste}$
und $MG_{Silikonrest} = n \cdot 74{,}1 + 132{,}2$
sowie $MG_{Polytherreste} = 2 \cdot (m \cdot 14 + 16 + x \cdot 44 + y \cdot 58 + z)$ mit z = 1, 15, 29
enthalten. Über Silikonöl-in-Wasser-Emulsionen wird hingegen nichts offenbart.

Es hat sich für den Fachmann nicht vorhersehbar herausgestellt, daß
kosmetische und/oder dermatologische Silikon-in-Wasser-Emulsionen
mit einem Anteil an Silikonölen in der Fettphase von mehr als 50 Gew.% die polyethermodifizierte Polysiloxane enthalten, dadurch gekennzeichnet, daß als polyethermodifiziertes Polysiloxan Bis-PEG PPG-16/16 PEG/PPG-16/16 Dimethicone enthalten ist, den Mängeln des Standes der Technik abhelfen.

Dabei stabilisiert der besondere Emulgator die ohne seinen Einsatz teilinvertierten erfindungsgemäßen W/Si-Si/W-Emulsionen, die nicht stabil sind, zu stabilen Si/W-Emulsionen. Die erfindungsgemäßen Emulsionen zeichnen sich durch ein neuartiges Hautgefühl aus, daß sich als langanhaltend, seidig und trocken beschreiben läßt. Dabei kann das oft auftretende Klebrigkeitsgefühl auch ohne den Einatz sehr großer Silikonmengen vermieden werden. Zugleich führt die geringere Menge an Silikonöl dazu, daß sich auch höhere Mengen an Lichtschutzfiltern einarbeiten lassen, da diese in Silikonölen schlecht löslich sind. Folglich können erfindungsgemäße Emulsionen mit besonders hohen Lichtschutzfaktoren formuliert werden. Vorteilhaft lassen sich auch dünnflüssige, sprühbare Emulsionen formulieren, die auch ohne den Einsatz von Fettalkoholen auskommen und dennoch langzeitlagerstabil sind. Im Gegensatz zu bekannten PIT-Emulsionen, bei deren Formulierung eine verhältnismäßig geringe Auswahl an Rohstoffen zur Verfügung steht, kann im Falle der erfindungsgemäßen Si/W-Emulsionen auf einen breiteren Fundus an Rohstoffen zurückgegriffen werden, so daß nahezu jede gewünschte Sensorik erzielt werden kann. Dabei können kosmetische Wirkstoffe in vorteilhafter Weise stabil formuliert werden: hydrophile Wirkstoffe bleiben in der Wasserphase und zeigen keine Tendenz sich zum Teil in die Silikonphase umzuverteilen; hierdurch wird eine sehr schnelle Freisetzung des Wirkstoffes und damit eine gute Verfügbarkeit auf der Haut gewährleistet. Umgekehrt erfahren lipophile Wirkstoffe eine Stabilisierung dadurch, dass sie keine Tendenz zeigen, die Silikonölphase zu verlassen bzw. sich an der Grenzschicht Emulgator/Wasserphase anzureichern; hierdurch wird ein Abbau durch Kontakt mit der Wasserphase minimiert und der Wirkstoff optimal vor Wechselwirkungen mit anderen (hydrophilen) Wirkstoffen in der Wasserphase bewahrt. Analog führt die geringe Wasseraufnahmefähigkeit der Silikonöle zu einer verbesserten Wasserfestigkeit beispielsweise im Falle von Sonnenschutzzubereitungen. Auch der Verdünnung der auf die Haut aufgetragenen Zubereitung durch die Schweißsekretion des Körpers wird vermindert.

Es ist bevorzugt, wenn der Gehalt an polyethermodifizierten Polysiloxanen 0,25 bis 15 Gew.%, bezogen auf das Gesamtgewicht der Emulsion beträgt.

Weiterhin ist es bevorzugt, wenn die Emulsion einen Gehalt an mindestens einem nichtionischen Emulgator aufweist.

Besonders bevorzugt ist es, wenn die nichtionischen Emulgatoren gewählt werden aus der Gruppe der Glycerylstearate, Polyethylenglykole und/oder der Alkylpolyglycoside.

Ganz besonders bevorzugt ist es, wenn die nichtionischen Emulgatoren gewählt werden aus der Gruppe der Glycerylstearatcitrat, Polyethylenglykol 40, Polyethylenglykol-100, Polyglycerin-3-methylglucosedistearat, Glycerylstearat.

Weiterhin ist es bevorzugt, wenn die Emulsion einen Gehalt an mindestens einem anionischen Emulgator aufweist.

Besonders bevorzugt ist es, wenn die anionischen Emulgatoren gewählt werden aus der Gruppe Trilaureth-4-phosphat, Cetylphosphat, Natriumcetearylsulfat, Salze der Stearinsäure.

Dabei ist es besonders bevorzugt, wenn der Gehalt an nichtionischen und/oder anionischen Emulgatoren 0,01 bis 5 Gew.% bezogen auf das Gesamtgewicht der Emulsion beträgt.

Ganz besonders bevorzugt ist es, wenn das Gewichtsverhältnis von nichtionischen und/oder anionischen Emulgatoren zu polyethermodifizierten Polysiloxanen 0,001 bis 20 beträgt.

Darüber hinaus ist es bevorzugt, wenn die Emulsion einen Gehalt an Tensiden gewählt aus der Gruppe der tensidischen Betaine, Alkylpolyglycoside, Aminosäurederivate oder quaternäre Ammoniumverbindungen aufweist.

Der Einsatz dieser Tenside ist unbedingt erforderlich, wenn die PIS-Technik zur Herstellung der erfindungsgemäßen Emulsionen zum Einsatz kommen soll, mit der besonders elegant vorteilhafte Tröpfchengrößenverteilungen erzielt werden können. Aber auch ohne Einsatz der PIS-Technik zur Herstellung der erfindungsgemäßen Emulsionen ist ein Gehalt an tensidischen Betainen, Alkylpolyglycosiden, Aminosäurederivaten oder quaternäre Ammoniumverbindungen von Vorteil, da die Stabilität der Emulsionen durch diesen stark verbessert wird.

Dabei ist es besonders bevorzugt, wenn als tensidischen Betaine

Alkylopolybetaine der allgemeinen Formel

(I)

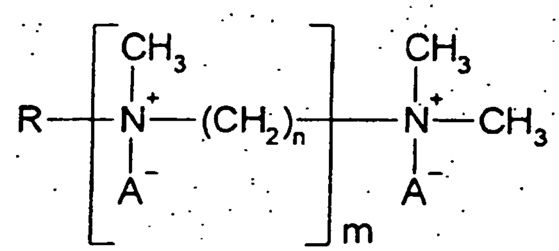

mit

R = Alkyl (C12-18),

A = -CH2CHOHCH2SO3- (a) oder -CH2COO- (b)

m = 1-3,

Kokosfettsäureamidopropyl Betain ( Cocamidopropyldimethylglycin, CAS 61789-40-0),

Weizenkeimfettsäureamidopropylbetain (INCI: Wheat Germamidopropyl betaine im Handel erhältlich unter der Bezeichnung Mackam WGB),

Kokosnußfettsäure und Ölsäureamidopropyl Betaine (INCI: Coco/Oleamidopropyl Betaine CAS 86438-79-1,im Handel erhältlich unter der Bezeichnung Mirataine COB, Fa. Miranol),

Dimethicon Propyl PG-Betain ( im Handel erhältlich unter der Bezeichnung Abil B® 9950),

Isostearamidopropyl Betain (im Handel erhältlich unter der Bezeichnung Schercotaine IAB),

Palmitamidopropyl Betaine (N-(Carboxymethyl)-N,N-dimethyl-3[(1-oxohexadecyl)amino]-1-propanamoniumhydroxid, CAS 32954-43-1, im Handel erhältlich unter der Bezeichnung Incronam P-30),

Stearamidopropyl Betaine (Stearoylamidpropyldimethylglycin),

Sulfobetaine der Formel

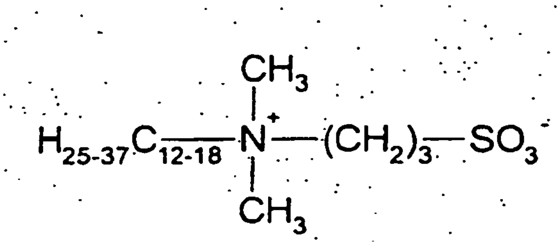

sowie Capryl/Capramidopropyl Betain (im Handel erhältlich unter der Bezeichnung Tego Betain 810 (Goldschmidt), ganz besonders bevorzugt Capryl/Capramidopropyl Betain verwendet werden.

Dabei ist es besonders bevorzugt, wenn als Alkylpolyglycoside Verbindungen der Formel

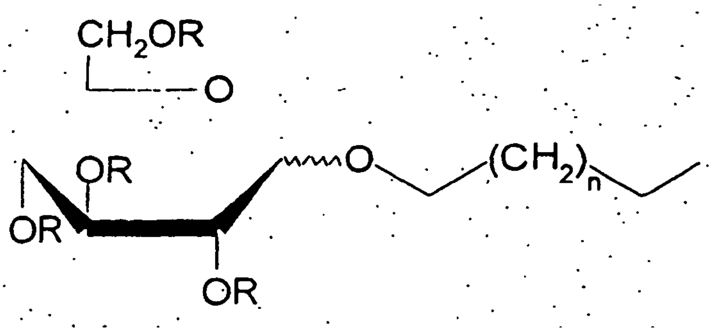

wobei R = H oder Glucosyl und n = 5-13 ist, eingesetzt werden.

Ganz besonders bevorzugtes Alkylpolyglycoside sind Caprylyl/Capryl Glucoside der Formel

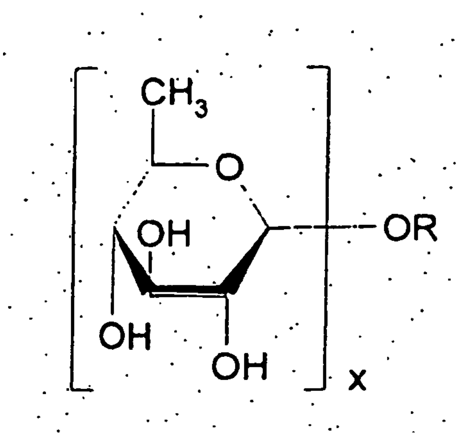

(I)

wobei R ein Gemisch aus Caprylyl- u. Caprylresten ist (im Handel erhältlich unter der Bezeichnung Triton CG-IIO), Cetearyl Glucoside (Ketostearylether der Glukose; im Handel erhältlich unter der Bezeichnung Montanol 68), Decylglucosid (im Handel erhältlich unter der Bezeichnung Plantaren 2000 ),

Laurylglucosid (im Handel erhältlich unter der Bezeichnung Plantaren 1200 ).

Dabei ist es besonders bevorzugt, wenn als Aminosäurederivate N-Cocoyl-L-glutaminsäure (COCOYL GLUTAMIC ACID, im Handel erhältlich unter der Bezeichnung Amisoft CA (Ajinomoto)),
Dinatrium L-N-Cocoylglutamat (INCI: DISODIUM COCOYL GLUTAMATE, im Handel erhältlich unter den Bezeichnungen Amisoft ECS-22 (Ajinomoto), Amisoft CS-22 (Ajinomoto)),
Dinatrium Lauroylglutamat (INCI: DISODIUM LAUROYL GLUTAMATE (im Handel erhältlich unter der Bezeichnung Amisoft LS-22 (Ajinomoto)),
Dinatrium N-(1-Oxooctadecyl)-L-Glutamat (INCI: DISODIUM STEAROYL GLUTAMATE, im Handel erhältlich unter der Bezeichnung Amisoft HS-21(P) (Ajinomoto),
Kalium N-Cocoacyl-L-glutamat (INCI: POTASSIUM COCOYL GLUTAMATE, im Handel erhältlich unter den Bezeichnungen Amisoft CK-11 (Ajinomoto), Amisoft CK-22 (Ajinomoto),
Kalium N-Lauroyl-L-glutamate (INCI: Potassium LAUROYL GLUTAMATE, im Handel erhältlich unter der Bezeichnung Amisoft LK-11 (Ajinomoto),
Triethanolamin N-Cocoyl-L-glutamat (INCI TEA-Cocoyl glutamate, im Handel erhältlich unter der Bezeichnung Amisoft CT-12 (Ajinomoto),
Natrium N-(1-Oxooctadecyl)-L-Glutamat (INCI: Natrium Stearoylglutamat, im Handel erhältlich unter den Bezeichnungen Amisoft HS-11P (Ajinomoto) und Amisoft GS-11P(33377) (Ajinomoto)),
DL-Pyrrolidonecarbonsäuresalz des L-Cocoylargininethylesters (INCI: PCA ETHYL COCOYL ARGINATE, im Handel erhältlich unter der Bezeichnung CAE (Ajinomoto)),
Triethanolamin N-Cocoyl-L-alaninat (INCI TEA-Cocoyl alaninate, im Handel erhältlich unter der Bezeichnung Amilite ACT-12 (Ajinomoto)),
Natrium N-Kokosacylglycinat (INCI: Sodium Cocooyl Glycinate, im Handel erhältlich unter den Bezeichnungen Amilite GCS-12 (Ajinomoto) und Amilite GCS-11F (Ajinomoto),
N-(Carboxymethyl)-N,N-Bis(2-Hydroxyethyl)-1-Octadecanaminiumhydroxide (INCI: Dihydroxyethyl Stearyl Glycinate),

Natrium N-Cocoyl-L-glutamat (INCI: Natrium Cocoyl Glutamate, im Handel erhältlich unter den Bezeichnungen Amisoft CS-11 (Ajinomoto), Hostapon CCG (Clariant GmbH), Hostapon KCG (Clariant GmbH (Surfactants, Personal Care)), Amisoft CS-22(32668) (Ajinomoto), Amisoft GS-11(32669) (Ajinomoto), Amisoft GS-11P(32670) (Ajinomoto), Elespher Vitaplex Hydro(32671) (Serobiologiques), eingesetzt werden.
Ein ganz besonders bevorzugtes Aminosäurederivat ist Natrium N-Cocoyl-L-glutamat.
Dabei ist es besonders bevorzugt, wenn als quaternäre Ammoniumverbindungen Coco Alkyl Ethyldimethylammonium Ethyl Sulfate (INCI: Coco-Ethyldimonium-Ethosulfate, im Handel erhältlich unter der Bezeichnung Dextrol AS-150 (Dexter)),
[2-[[2-[(2-Carboxyethyl)(2-Hydroxyethyl)Amino]Ethyl]Amino]-2-Oxoet, INCI: Cocobetainamido Amphopropionate, im Handel erhältlich unter der Bezeichnung REWOTERIC QAM 50 (Witco Surfactants GmbH)),
Cocoyltrimethylammoniumchlorid (INCI: Cocoyltrimonium Chloride, im Handel erhältlich unter den Bezeichnungen Arquad C-33W (Akzo Nobel), Arquad C-50(9269) (Akzo Nobel), Masil EM 930 C(9270) (BASF), Servamine KAC 412(9271) (Servo Delden), Solvariane(9272) (Wackherr), Di-C12-15-Alkyldimethylammoniumchlorid (INCI: Di-C12-15-Alkyldimoniumchloride, im Handel erhältlich unter den Bezeichnungen Carsoquat 457 E (Lonza Inc./Lonza Ltd.), Carsoquat 457 I (Lonza Inc./Lonza Ltd.), Carsoquat 457 P (Lonza Inc./Lonza Ltd.),
Quaternium-18 (im Handel erhältlich unter den Bezeichnungen AEC Quaternium-18 (A & E Connock), Arquad HC (Akzo

Nobel Surface Chemistry), Kemamine Q-9702C (Witco), Radiaquat 6442 (Fina Chemicals), Varisoft DHT (Witco), Varisoft 442 100P (Witco), Arquad 2HT-75(30273) (Akzo Nobel)),
Quaternium-26 (im Handel erhältlich unter der Bezeichnung Ceraphyl 65 (ISP Van Dyk), Incroquat 26 (Croda, Inc.)),
Talgtrimethylammoniumchlorid (INCI: Tallowtrimonium Chloride, im Handel erhältlich unter der Bezeichnung Arquad T-30 (Akzo Nobel Surface Chemistry), Varisoft 471 (Witco), Arquad T-50(34946) (Akzo Nobel), Dow Corning 929 Cationic Emulsion(34947) (Dow Corning), Dow Corning 1669 Cationic Emulsion(34948) (Dow Corning), Hansicone E-2153 (34949) (Hansotech)) verwendet werden.

Ganz besonders bevorzugt sind erfindungsgemäße Silikon-in-Wasser-Emulsionen, die erhältlich sind durch Hochdruckhomogenisierung, wobei die Emulsion einer Hochdruckhomogenisierung bei 30 bis 1500 bar, besonders bevorzugt bei 750 bar unterworfen wird.

Weiter ganz besonders bevorzugt sind erfindungsgemäße Silikon-in-Wasser-Emulsionen, die erhältlich sind durch Anwendung der PIS-Technik gekennzeichnet durch

(a) Herstellung einer tensidfreien Wasser-in Silikon-Emulsion bei Temperaturen oberhalb 60°C, besonders bevorzugt oberhalb 50°C,
wobei die tensidfreie Wasser-in Silikon-Emulsion

- (a1) mehr als 50 Gew.% Silikonölen in der Fettphase,
- (a2) polyethermodifizierte Polysiloxane,
- (a3) mindestens einen nichtionischen Emulgator umfasst,

(b) Temperieren auf Temperaturen unterhalb 60°C, besonders bevorzugt unterhalb 50°C,

(c) Zugabe von weniger als 5 Gew.%, besonders bevorzugt weniger als 2 Gew.% Tensiden gewählt aus der Gruppe der tensidischen Betaine, Alkylpolyglycoside, Aminosäurederivate oder quaternäre Ammoniumverbindungen, um eine Phasenumkehr zu einer Silikon-in Wasser-Emulsion zu erreichen.

Schließlich ist es ganz besonders bevorzugt, wenn das Zahlenmittel der Tröpfchengröße. weniger als 1 µm, besonders bevorzugt 50 bis 300 nm, ganz besonders bevorzugt 70 bis 100 nm beträgt.

Vorteilhaft lassen sich sehr feinteilige erfindungsgemäße Emulsionen herstellen unter Einbeziehung eines Verfahrens der Tropfenzerkleinerung. Dabei wird im allgemeinen die Tröpfchengrößenverteilung vereinheitlicht. Dies kann in Homogenisatoren durch Energieeintrag in das betreffende disperse System erfolgen. Je nach Bauart der Homogenisators können unterschiedliche spezifische Energieeinträge in das zu homogenisiernde Gut realisiert werden. Üblicherweise kommen Homogenisatoren zum Einsatz, die nach dem Prinzip Rotor-Stator aufgebaut sind. Erheblich höhere Energieeinträge lassen sich durch Hochdruckhomogenisierung erzielen. Dabei wird das zu homogenisiernde Fluid mit einer Hochdruckpumpe verdichtet und anschließend durch ein Homogenisierventil entspannt. Der Druckabfall über das Homogenisierventil beträgt üblicherweise 30 bis 1500 bar, ausnahmsweise bis 2000 bar. Während des Entspannungsvorganges treten innerhalb des Fluides hohe Beschleunigungen und Turbulenzen auf, die eine Feinzerkleinerung der Tröpfchen bewirken. So können Tröpfchengrößen unterhalb von 1 µm, gewöhnlich 50 bis 200 nm erreicht werden. Um erfindungsgemäße Emulsionen herstellen zu können ist es besonders bevorzugt, wenn eine Hochdruckhomogenisierung bei 30 bis 1500 bar, besonders bevorzugt bei 750 bar durchgeführt wird. So lassen sich besonders gut die erfindungsgemäßen Tröpfchengrößen erreichen.

Besipielhafte Herstellung einer Si/W-Emulsion nach dem Hochdruckhomogenisationsverfahren:

1. Herstellung einer Emulsion im 2,5 to Beco-Mischer:
Phase A bestehend aus Wasser, Glycerin, Methylparaben und Propylparaben und Panthenol wurden im Phasenkessel A vorgelegt und auf 75°C erhitzt. Die Phase B, bestehend aus Bis-PEG/PPG-16/16 PEG/PPG16/16 Dimethicone, Caprylic/Caprictriglycerid, Dimethicon, etc. wird im Phasenkessel B auf 80°C erhitzt und innerhalb von 5 Minuten in den Phasenkessel A gepumpt. Währendessen läuft der Umlaufhomogenisator mit 14 m/s und es wird ein Vakuum von -0.4 bar angelegt, das Rührwerk läuft mit ca. 30 U/min. Nach dem erfolgreichen Emulgieren wird zuerst Phase C, bestehend aus Cyclomethicon, unter Rühren und halten des Vakuums über den statischen Homogenisator eingesaugt. Jetzt erfolgt die Kühlung mit 15°C kaltem Wasser und bei 35°C erfolgt die Zugabe der Parfumphase.

2. Nach weiteren 5 Minuten wird der gesamte Ansatz in einem Hochdruckhomogenisator bei ca. 750 bar homogensiert.

3. Dann folgt eine weitere Abkühlung auf 30°C und das Produkt ist zur Abfüllung bereit.

Besonders vorteilhaft zur Herstellung erfindungsgemäßer Emulsionen ist das im folgenden beschriebene so genannte PIS-Verfahren. Es ist bekannt, daß bestimmte hydrophile Emulgatoren wie typischerweise Ethylenglykoldialkylether beispielsweise Polyoxyethylen(20)cetylstearylether (Ceteareth-20) bei steigender Temperatur ihr Löslichkeitsverhalten von wasserlöslich zu fettlöslich ändern. Der Temperaturbereich, in dem die Emulgatoren ihre Löslichkeit geändert haben, wird Phaseninversionstemperaturbereich (PIT) genannt. Diese Eigenschaft ist Grundlage für ein Herstellungsverfahren für Emulsionen, der sogenannten PIT-Technik. Dabei wird die Erscheinung verwendet, daß emulgatorstabilisierte O/W oder Si/W-Emulsionen beim Überschreiten der Phaseninversionstemperatur eine in der Regel reversible Phaseninversion erleiden, also die innere Phase zur äußeren wird. Man erhält zunächst also eine W/O- oder W/Si--Emulsion. Kühlt man die invertierte Emulsion wieder unter die Phaseninversionstemperatur ab, so können O/W oder Si/W-Emulsionen mit besonders niedrigen Tröpfchengrößen entstehen.

Formulierungen, die überwiegend un- bzw. mittelpolare Lipide, sie polyethermodifizierte Polysiloxane und Hilfstoffe enthalten, bilden ebenso zuerst eine metastabile W/O- oder W/Si-Emulsion aus. Diese W/O- oder W/Si-Emulsion zeichnet sich durch eine sehr kleine Tröpfchengröße aus und ist nur zeitlich begrenzt stabil (max. 3 Monate bei Raumtemperatur). Nach Herstellung dieser W/O- oder W/Si-Emulsion setzt man unterhalb von 50°C erfindungsgemäße Tenside in einem Konzentrationsbereich von unter 5 Gew.%, besonders bevorzugt unter 2 Gew.%, bezogen auf die Gesamtformulierung, hinzu und erhält eine Phaseninvertierung zur O/W oder Si/W-Emulsion. Durch diese Phaseninversion konserviert man die kleine Tröpfchengröße und erhält eine langzeitstabile O/W oder Si/W-Emulsion. Diese Inversion durch den Zusatz von Tensiden kann auch erhalten werden, wenn die Lipidphase überwiegend aus lineare bzw. cyclischen Silikonölen besteht. Dann wird zuerst eine metastabile Wasser-in-Silikon-Emulsion gebildet, die durch Tensidzusatz, invertiert und eine langzeitstabile Silikon-in-Wasser-Emulsion ausbildet.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagespflegeprodukten oder Make-up-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Dementsprechend enthalten die Zubereitungen im Sinne der vorliegenden Erfindung vorzugsweise mindestens eine UV-A-, UV-B- und/oder Breitbandfiltersubstanz und/oder mind. ein UV-Licht reflektierendes und/oder absorbierendes anorganisches Pigment. Die Formulierungen können, obgleich nicht notwendig, gegebenenfalls auch ein oder mehrere organische und/oder anorganische Pigmente als UV-Filtersubstanzen enthalten, welche in der Wasser- und/ oder der Ölphase und/oder der Emulsionsphase vorliegen können.

Die erfindungsgemäßen Zubereitungen können ferner vorteilhaft auch in Form von sogenannten ölfreien kosmetischen oder dermatologischen Zubereitungen vorliegen, wobei die natürlichen Öle und die synthetischen Öle durch bei Raumtemperatur flüssige UV-Filter und/oder Silikonderivate ersetzt werden.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Ethylhexyl Salicylate) und Ester der Zimtsäure, vorzugsweise 4-Methoxyzimtsäure(2-ethylhexyl)ester (2-Ethylhexyl-4-methoxycinnamat, INCI: Ethylhexyl Methoxycinnamate) und 4-Methoxyzimtsäureisopentylester (Isopentyl-4-methoxycinnamat, INCI: Isoamyl p-Methoxycinnamate) und polymere UV-Filter wie das (3-(4-(2,2-bis-Ethoxycarbonylvinyl)-phenoxy) propenyl)- methylsiloxan/Dimethylsiloxan Copolymer, welches beispielsweise bei Hoffmann-La Roche unter der Handelsbezeichnung Parsol SLX erhältlich ist.
Bevorzugte anorganische Pigmente sind Metalloxide und/oder andere in Wasser schwerlösliche oder unlösliche Metallverbindungen, insbesondere Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$), Cers (z. B. $Ce_2O_3$), Mischoxide der entsprechenden Metalle sowie Abmischungen aus solchen Oxiden sowie das Sulfat des Bariums ($BaSO_4$).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid ($Al_2O_3$), Aluminiumhydroxid $Al(OH)_3$, bzw. Aluminiumoxidhydrat (auch: Alumina, CAS-Nr.: 1333-84-2), Natriumhexametaphosphat $(NaPO_3)_6$, Natriummetaphosphat $(NaPO_3)_n$, Siliciumdioxid ($SiO_2$) (auch: Silica, CAS-Nr.: 7631-86-9), Bariumsulfat ($BaSO_4$) oder Eisenoxid ($Fe_2O_3$). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| 1.1.1 Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |
| MZ-505S | 5% Methicone | Tayca Corporation |

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul $TiO_2$) | Octyltrimethylsilan | Degussa |

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:

- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazole Tetrasulfonate (CAS-Nr.: 180898-37-7), welches beispielsweise unter der Handelsbezeichnung Neo Heliopan AP bei Haarmann & Reimer erhältlich ist;

- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;

- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;

- Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

- Hydroxybenzophenon-Derivate, wie z.B. 2-(4-Diethylamino-2-hydroxybenzoyl)-benzoic acid hexylester, welches beispielsweise von der Firma BASF unter dem Handelsnamen Uvinul® A Plus erhältlich ist.

- Benzoxazol-Derivate, wie z.B. das 2,4-bis-[5-1(dimethylpropyl)benzossazol-2-yl-(4-phenyl)-imino]-6-(2-ethylhexyl)-imino-1,3,5-triazin (CAS-Nr.: 288254-16-0), welches beispielsweise von der Firma 3V Sigma erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.
Vorteilhafte Breitbandfilter oder UV-B-Filtersubstanzen sind beispielsweise Triazinderivate, wie z.B.

- 2,4-Bis-{[4-(2-Ethyl-hexyloxy)-2-hydroxy]-phenyl}-6-(4-methoxyphenyl)-1,3,5-triazin (INCI: Bis-Ethylhexyloxyphenol Methoxyphenyl Triazin), welches unter der Handelsbezeichnung Tinosorb® S bei der CIBA-Chemikalien GmbH erhältlich ist;

- Diethylhexylbutylamidotriazon (INCI: Diethylhexyl Butamido Triazone), welches unter der Handelsbezeichnung UVASORB HEB bei Sigma 3V erhältlich ist;

- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird.

Ein vorteilhafter Breitbandfilter im Sinne der vorliegenden Erfindung ist auch das 2,2'-Methylenbis-(6-(2H-benzotriazol-2-yl)-4-(1,1,3,3-tetramethylbutyl)-phenol) Cas-Nr.: 103597-45-1, welches unter der Handelsbezeichnung. Tinosorb® M bei der CIBA-Chemikalien GmbH erhältlich ist.
Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches unter der Handelsbezeichnung Mexoryl® XL bei der Fa. Chimex erhältlich ist.
Die weiteren UV-Filtersubstanzen können öllöslich oder wasserlöslich sein.
Vorteilhafte öllösliche UV-B- und/oder Breitband-Filtersubstanzen im Sinne der vorliegenden Erfindung sind z. B.:

- ■ 3-Benzylidencampher-Derivate; vorzugsweise 3-(4-Methylbenzyliden)campher, 3-Benzylidencampher;

- ■ 4-Aminobenzoesäure-Derivate, vorzugsweise 4-(Dimethylamino)-benzoesäure(2-ethylhexyl)ester, 4-(Dimethylamino)benzoesäureamylester;

- ■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon

- ■ sowie an Polymere gebundene UV-Filter.

- ■ 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Besonders vorteilhafte Zubereitungen im Sinne der vorliegenden Erfindung, die sich durch einen hohen bzw. sehr hohen UV-A- und/oder UV-B-Schutz auszeichnen, enthalten neben der oder den erfindungsgemäßen Filtersubstanz (en) bevorzugt ferner weitere UV-A- und/oder Breitbandfilter, insbesondere Dibenzoylmethanderivate [beispielsweise das 4-(tert.-Butyl)-4'-methoxydibenzoylmethan], Phenylen-1,4-bis-(2-benzimidazyl)-3,3'-5,5'-tetrasulfonsäure und/oder ihre Salze, das 2-(4-Diethylamino-2-hydroxybenzoyl)-benzoic acid hexylester und/oder das 2,4-Bis-{[4-(2-Ethyl-hexyloxy)-2-hydroxy]-phenyl}-6-(4-methoxyphenyl)-1,3,5-triazin und/oder das 2,4-bis-[5-1(dimethylpropyl)benzossazol-2-yl-(4-phenyl)-imino]-6-(2-ethylhexyl)-imino-1,3,5-triazine, jeweils einzeln oder in beliebigen Kombinationen miteinander.
Die Liste der genannten UV-Filter, die im Sinne der vorliegenden Erfindung eingesetzt werden können, soll selbstverständlich nicht limitierend sein.
Die erfindungsgemäß vorteilhaften UV-Lichtschutzfilter werden bevorzugt in einer Konzentration von 0,1 bis 30 Gewichts-%, insbesondere in einer Konzentration von 0,5 bis 15 Gewichts-%, bezogen auf das Gesamtgewicht der Formulierung, eingesetzt. Erfindungsgemäß können die kosmetischen und/oder dermatologischen Lichtschutzformulierungen wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.
Zur Anwendung werden die kosmetischen und dermatologischen Zubereitungen erfindungsgemäß in der für

Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoëharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 10 Gew.-%, besonders bevorzugt 0,05 - 7 Gew.-%, insbesondere 0,1 - 5 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 5 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 5 Gew.-%, bezogen auf das Gesamtgewicht der Formulierung, zu wählen.

Darüber hinaus eignen sich ausgewählte erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, insbesondere vorteilhaft zur Prophylaxe und Behandlung kosmetischer oder dermatologischer Hautveränderungen, wie sie z. B. bei der Hautalterung (z.B. Falten und Fältchen) auftreten. Weiterhin vorteilhaft eignen sie sich gegen das Erscheinungsbild der trockenen bzw. rauhen Haut.

Sofern α-Glycosylrutin das Antioxidants darstellt, ist vorteilhaft, deren jeweilige Konzentrationen aus dem Bereich von 0,001 - 5 Gew.-%, bezogen auf das Gesamtgewicht der Formulierung, zu wählen.

Eine als zweite, eigenständige Wasserphase vorliegende wässrige Phase bzw. die Wasserphase der O/W oder Si/W-Emulsion der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. NOVEON], beispielsweise Carbopole der Typen 980, 981, 1382, 2984, 5984, ETD 2020, ETD 2050, Ultrez 10, jeweils einzeln oder in Kombination. Die erfindungsgemäßen Zubereitungen können ferner vorteilhaft auch Selbstbräunungssubstanzen enthalten, wie beispielsweise Dihydroxyacteon und/oder

Melaninderivate in Konzentrationen von 1 Gew.-% bis zu 10 Gew.-%, bezogen auf das Gesamtgewicht der Zubereitung.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Eine zweite, als eigenständige Ölphase vorliegende lipophilen Phase und/oder die Ölphase der O/W oder Si/W-Emulsion der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner können die Ölphasen vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, $C_{12-13}$-Alkyllactat, Di-$C_{12-13}$-Alkyltartrat, Triisostearin, Dipentaerythrityl Hexacaprylat/Hexacaprat, Propylenglykolmonoisostearat, Tricaprylin, Dimethylisosorbid zu wählen. Es ist insbesondere vorteilhaft, wenn die Ölphase der erfindungsgemäßen Formulierungen einen Gehalt an $C_{12-15}$-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Hallbrite TQ oder Corapan TQ von H&R*).

Auch beliebige Abmischungen solcher Öl- und Wachskomponenten sind vorteilhaft im Sinne der vorliegenden

Erfindung einzusetzen.

Ferner können die Ölphasen ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft können die Ölphasen ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen Silicium-Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, PhenylGruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen

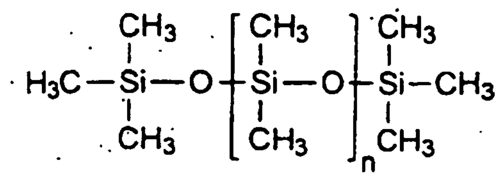

werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die erfindungsgemäßen Zubereitungen können ferner vorteilhaft eine oder mehrere Substanzen aus der folgenden Gruppe der Siloxanelastomere enthalten, beispielsweise um die .Wasserfestigkeit und/oder den Lichtschutzfaktor der Produkte zu steigern:

(a) Siloxanelastomere, welche die Einheiten $R_2SiO$ und $RSiO_{1,5}$ und/oder $R_3SiO_{0,5}$ und/oder $SiO_2$ enthalten, wobei die einzelnen Reste R jeweils unabhängig voneinander Wasserstoff, $C_{1-24}$-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten $R_2SiO$ zu $RSiO_{1,5}$ aus dem Bereich von 1 : 1 bis 30 : 1 gewählt wird;

(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,

wobei die verwendeten Mengenateile so gewählt werden, daß die Menge des Wasserstoffes des Organopolysiloxans (1) oder der ungesättigten aliphatischen Gruppen des Organopolysiloxans (2)

- im Bereich von 1 bis 20 mol-% liegt, wenn das Organopolysiloxan nicht cyclisch ist und
- im Bereich von 1 bis 50 mol-% liegt, wenn das Organopolysiloxan cyclisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.
Ganz besonders bevorzugt ist es, wenn das Siloxanelastomer in Kombination mit unverzweigten bei Raumtemperatur flüssigen oder pastösen Silikonölen oder cyclischen Silikonölen oder deren Gemischen verwendet wird. Insbesondere vorteilhaft sind Organopolysiloxanelastomere mit der INCI-Bezeichnung Dimethicone / Polysilicone-11, ganz besonders die von der Grant Industries Inc. erhältlichen Gransil-Typen GCM, GCM-5, DMG-6, CSE Gel, PM-Gel, LTX, ININ Gel, AM-18 Gel und/oder DMCM-5.
Ganz außergewöhnlich bevorzugt ist es, wenn das Siloxanelastomer in Form eines Gels aus Siloxanelastomer und einer Lipidphase verwendet wird, wobei der Gehalt des Siloxanelastomers in dem Gel 1 bis 80 Gew.-%, bevorzugt 0,1 bis 60 Gew.-% beträgt, jeweils bezogen auf das Gesamtgewicht des Gels.
Es ist vorteilhaft im Sinne der vorliegenden Erfindung, die Gesamtmenge der Siloxanelastomere (Aktivgehalt) aus dem Bereich von 0,01 bis 10 Gew.-%, vorteilhaft von 0,1 bis 5 Gew.-%, jeweils bezogen auf das Gesamtgewicht der Formulierung, zu wählen.

Die erfindungsgemäßen kosmetischen und dermatologischen Zubereitungen können Farbstoffe und/oder Farbpigmente enthalten, insbesondere wenn sie in Form von dekorativen Kosmetika vorliegen. Dabei können erfindungsgemäß vorteilhaft die einzelnen Phasen der Zubereitung unterschiedlich gefärbt sein. Auch Ausführungsformen der Erfindung, bei denen lediglich eine der beiden Phasen gefärbt ist, sind erfindungsgemäß vorteilhaft. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise.Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus dem *Rowe Colour Index, 3. Auflage, Society of Dyers and Colourists, Bradford, England, 1971* zu wählen.

Sofern die erfindungsgemäßen Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigodisulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:

1. Natürliche Perlglanzpigmente, wie z. B.

   - "Fischsilber" (Guanin/Hypoxanthin-Mischkristalle aus Fischschuppen) und
   - "Perlmutt" (vermahlene Muschelschalen)

2. Monokristalline Perlglanzpigmente wie z. B. Bismuthoxychlorid (BiOCl)

3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| **Gruppe** | Belegung / Schichtdicke | Farbe |
|---|---|---|
| Silberweiße Perlglanzpigmente | $TiO_2$: 40 - 60 nm | silber |

(fortgesetzt)

| **Gruppe** | Belegung / Schichtdicke | Farbe |
|---|---|---|
| Interferenzpigmente | $TiO_2$: 60 - 80 nm | gelb |
| | $TiO_2$: 80 - 100 nm | rot |
| | $TiO_2$: 100 - 140 nm | blau |
| | $TiO_2$: 120 - 160 nm | grün |
| Farbglanzpigmente | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| Kombinationspigmente | $TiO_2$ / $Fe_2O_3$ | Goldtöne |
| | $TiO_2$/$Cr_2O_3$ | grün |
| | $TiO_2$ / Berliner Blau | tiefblau |
| | $TiO_2$/Carmin | rot |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO2 und Fe2O3 beschichtete SiO2-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (Cl) Nummern 19140, 77007, 77289, 77491) vor.

Die Farbstoffe und Pigmente können sowohl einzeln als auch in Kombination vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), Iodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Erfindungsgemäß vorteilhaft ist ferner die Aufbewahrung der erfindungsgemäßen Zubereitung in klaren und/ oder durchscheinenden Verpackungsbehältnissen.

Die Viskositäten der erfindungsgemäßen Zubereitungen liegen vorteilhaft zwischen wässrig dünn (0-700 mPas) bis hin zu einer fließfähigen Konsistenz (700-4000 mPas). Die sehr dünnflüssigen Zubereitungen könnten in Behältnissen mit Pumpsystemen als Spray oder als Fluid eingesetzt werden.

Die erfindungsgemäßen Zubereitungen können erfindungsgemäß vorteilhaft als Salbe, Créme oder Lotion eingesetzt werden. Auch ist ihr Einsatz in Form eines Sprays, z.B. eines Pumpsprays erfindungsgemäß vorteilhaft, wobei die Zubereitungen vorteilhafterweise auch aufgeschäumt werden können.

Es ist ferner erfindungsgemäß besonders vorteilhaft, die erfindungsgemäßen Zubereitungen in transparenten und/oder transluzenten Verpackungen aufzubewahren und darzureichen.

Das Weglassen eines einzelnen Bestandteile beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.

Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden. Vielfalt an geeigneten Komponenten derartiger Zubereitungen für den Fachmann nicht unerwartet, so daß er weiß, daß bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

Beispiele

**Silikon in Wasser-Emulsion**

| | **1** | **2** | **3** | **4** | **5** |
|---|---|---|---|---|---|
| Bis-PEG/PPG-16/16 PEG/PPG16/16 Dimethicone, Caprylic/Caprictriglycerid | 1,0 | 2,0 | 8,0 | 3,0 | 5,0 |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Glycerin | 5,0 | 7,5 | 10,0 | 3,0 | 1,0 |
| Carbomer | --- | 0,1 | -- | 0,25 | 1,0 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| Natriumhydroxid 10%ig | --- | 0,1 | --- | 2,5 | 10,0 |
| modifizierte Stärke | --- | 2,5 | --- | 0,15 | -- |
| Wasser | ad 100 | ad 100 | ad 100 | ad 100 | ad 100 |

**Silikon in Wasser-Emulsion**

| | **6** | **7** | **8** | **9** | **10** |
|---|---|---|---|---|---|
| Bis-PEG/PPG-16/16 PEG/PPG16/16 Dimethicone, Caprylic/Caprictriglycerid | 1,0 | 2,0 | 8,0 | 3,0 | 5,0 |
| Cyclomethicon | 12,5 | 15 | 25,0 | 10,0 | 7,5 |

(fortgesetzt)

| | 6 | 7 | 8 | 9 | 10 |
|---|---|---|---|---|---|
| Dimethicon | 5,0 | 15,0 | 5,0 | 12,0 | 15,0 |
| Capryl/Capramidopropyl Betain | 0,15 | --- | 1,0 | --- | --- |
| Natrium Cocoylglutamat | --- | 1,0 | --- | --- | --- |
| Decylglucosid | --- | --- | ---- | 1,0 | 0,2 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Glycerin | 5,0 | 7,5 | 10,0 | 3,0 | 1,0 |
| Carbomer | --- | 0,1 | -- | 0,25 | 0,5 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Iodopropynylbutylcarbamat | --- | --- | 0,05 | --- | 0,1 |
| Natriumhydroxid 10%ig | --- | 0,1 | --- | 2,5 | 5,0 |
| modifizierte Stärke | --- | 2,5 | --- | 0,15 | -- |
| Wasser | ad 100 | ad 100 | ad 100 | ad 100 | ad 100 |

**Silikon in Wasser-Emulsion**

| | 11 | 12 | 13 | 14 | 15 |
|---|---|---|---|---|---|
| Bis-PEG/PPG-16/16 PEG/PPG16/16 Dimethicone, Caprylic/Caprictriglycerid | 1,0 | 2,0 | 8,0 | 3,0 | 5,0 |
| Cyclomethicon | 12,5 | 15 | 25,0 | 10,0 | 7,5 |
| Dimethicon | 5,0 | 15,0 | 5,0 | 12,0 | 15,0 |
| Quaternium-26 | 0,15 | --- | 1,0 | --- | --- |
| Quaternium-18 | -- | 0,3 | --- | 0,5 | --- |
| Capryl/Capramidopropyl Betain | --- | --- | --- | --- | 1,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Glycerin | 5,0 | 7,5 | 10,0 | 3,0 | 1,0 |
| Carbomer | --- | 0,1 | -- | 0,25 | 1,0 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Iodopropynylbutylcarbamat | --- | --- | 0,05. | --- | 0,1 |
| Natriumhydroxid 10%ig | --- | 0,1 | --- | 2,5 | 10,0 |
| modifizierte Stärke | --- | 2,5 | --- | 0,15 | -- |
| Wasser | ad 100 | ad 100 | ad 100 | ad 100 | ad 100 |

**Silikon in Wasser-Emulsion**

| | 16 | 17 | 18 | 19 | 20 |
|---|---|---|---|---|---|
| Bis-PEG/PPG-16/16 PEG/PPG16/16 Dimethicone, Caprylic/Caprictriglycerid | 1,0 | 2,0 | 8,0 | 3,0 | 5,0 |
| Cyclomethicon | 12,5 | 15 | 25,0 | 10,0 | 7,5 |
| Dimethicon | 5,0 | 15,0 | 5,0 | 12,0 | 15,0 |
| Capryl/Capramidopropyl Betain | 0,15 | --- | 1,0 | --- | 0,1 |
| Decylglucosid | -- | 0,3 | 0,2 | 0,5 | --- |
| Quaternium-18 | 0,15 | --- | --- | 0,5 | 1,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Glycerin | 5,0 | 7,5 | 10,0 | 3,0 | 1,0 |
| Carbomer | --- | 0,1 | -- | 0,25 | 1,0 |
| Panthenol | 0,5 | 1,0 | 0,75 | 0,25 | 0,1 |
| Parfum | q,s | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Iodopropynylbutylcarbamat | --- | --- | 0,05 | --- | 0,1 |
| Natriumhydroxid 10%ig | --- | 0,1 | --- | 2,5 | 0,5 |
| modifizierte Stärke | --- | 2,5 | --- | 0,15 | -- |
| Wasser | ad 100 | ad 100 | ad 100 | ad 100 | ad 100 |

**Silikon in Wasser-Emulsion**

| | 21 | 22 | 23 | 24 | 25 |
|---|---|---|---|---|---|
| Bis-PEG/PPG-16/16 PEG/PPG16/16 Dimethicone, Caprylic/Caprictriglycerid | 1,0 | 2,0 | 8,0 | 3,0 | 5,0 |
| Glycerylsterat | 1,0 | --- | --- | --- | 0,5 |
| Polyethylenglycol(40)stearat | --- | 2,0 | --- | --- | --- |
| Triglycerinmethylglucosedistearat | ---- | ---- | 3,0 | ---- | --- |
| Cetylphosphat | 0,5 | --- | --- | 1,5 | --- |
| Cyclomethicon | 12,5 | 15 | 25,0 | 10,0 | 7,5 |
| Dimethicon | 5,0 | 15,0 | 5,0 | 12,0 | 15,0 |
| Capryl/Capramidopropyl Betain | 0,15 | --- | 1,0 | --- | --- |
| Natrium Cocoylglutamat | --- | 1,0 | --- | --- | --- |
| Decylglucosid | --- | --- | ---- | 1,0 | 0,2 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Glycerin | 5,0 | 7,5 | 10,0 | 3,0 | 1,0 |
| Carbomer | --- | 0,1 | -- | 0,25 | 1,0 |

(fortgesetzt)

| | 21 | 22 | 23 | 24 | 25 |
|---|---|---|---|---|---|
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| Natriumhydroxid 10%ig | --- | 0,1 | --- | 2,5 | 1,0 |
| modifizierte Stärke | --- | 2,5 | --- | 0,15 | -- |
| Wasser | ad 100 | ad 100 | ad 100 | ad 100 | ad 100 |

**Patentansprüche**

1. Kosmetische und/oder dermatologische Silikon-in-Wasser-Emulsion mit einem Anteil and Silikohölen in der Fettphase von mehr als 50 Gew.%, die, polyethermodifizierte Polysiloxane enthält, **dadurch gekennzeichnet, daß** als polyethermodifiziertes Polysiloxan

   Bis-PEG/PPG-16/16 PEG/PPG16/16 (I)

   Dimethicone enthalten ist.

2. Silikon-in-Wasser-Emulsion nach Anspruch 1, **dadurch gekennzeichnet, daß** der Gehalt an polyethermodifizierten Polysiloxanen 0.25 bis 15 Gew.%, bezogen auf das Gesamtgewicht der Emulsion beträgt.

3. Silikon-in-Wasser-Emulsion nach einem der vorangehenden Ansprüche **dadurch gekennzeichnet, daß** sie einen Gehalt an mindestens einem nichtionischen Emulgator aufweist.

4. Silikon-in-Wasser-Emulsion nach einem der vorangehenden Ansprüche **dadurch gekennzeichnet, daß** die nichtionischen Emulgatoren gewählt werden aus der Gruppe der Glycerylstearate, Polyethylenglykole und/oder der Alkylpolyglycoside.

5. Silikon-in-Wasser-Emulsion nach einem der vorangehenden Ansprüche **dadurch gekennzeichnet, daß** die nichtionischen Emulgatoren gewählt werden aus der Gruppe der Glycerylstearatcitrat, Polyethylenglykol 40, Polyethylenglykol-100, Polyglycerin-3-methylglucosedistearat, Glycerylstearat.

6. Silikon-in-Wasser-Emulsion nach einem der vorangehenden Ansprüche **dadurch gekennzeichnet, daß** sie einen Gehalt an mindestens einem anionischen Emulgator aufweist.

7. Silikon-in-Wasser-Emulsion nach einem der vorangehenden Ansprüche **dadurch gekennzeichnet, daß** die anionischen Emulgatoren gewählt werden aus der Gruppe Trilaureth-4-phosphat, Cetylphosphat, Natriumcetearylsulfat, Salze der Stearinsäure.

8. Silikon-in-Wasser-Emulsion nach einem der vorangehenden Ansprüche **dadurch gekennzeichnet, daß** der Gehalt an nichtionischen und/oder anionischen Emulgatoren 0,01 bis 5 Gew.% bezogen auf das Gesamtgewicht der Emulsion beträgt.

9. Silikon-in-Wasser-Emulsion nach einem der vorangehenden Ansprüche **dadurch gekennzeichnet, daß** das Gewichtsverhältnis von nichtionischen und/oder anionischen Emulgatoren zu polyethermodifizierten Polysiloxanen 0,001 bis 20 beträgt.

10. Silikon-in-Wasser-Emulsion nach einem der vorangehenden Ansprüche **dadurch gekennzeichnet, daß** sie einen Gehalt an Tensiden gewählt aus der Gruppe der tensidischen Betaine, Alkylpolyglycoside, Aminosäurederivate

oder quaternäre Ammoniumverbindungen aufweist.

**11.** Silikon-in-Wasser-Emulsion nach einem der vorangehenden Ansprüche **dadurch gekennzeichnet, daß** als tensidischen Betaine Alkylopolybetaine. Kokosfettsäureamidopropyl Betain, Weizenkeimfettsäureamidopropylbetain, Kokosnußfettsäure und Ölsäureamidopropyl Betaine, Dimethicon Propyl PG-Betain, Isostearamidopropyl Betain, Palmitamidopropyl Betaine, Stearamidopropyl Betaine, Sulfobetaine der Formel $H_{25-37}C_{12-18}N^{+}$ $(CH_3)_2$-$(CH_2)_3$-$SO_3$, Capryl/Capramidopropyl Betain, besonders bevorzugt Capryl/Capramidopropyl Betain verwendet werden.

**12.** Silikon-in-Wasser-Emulsion nach einem der vorangehenden Ansprüche **dadurch gekennzeichnet, daß** als Alkylpolyglycoside Verbindungen der Formel

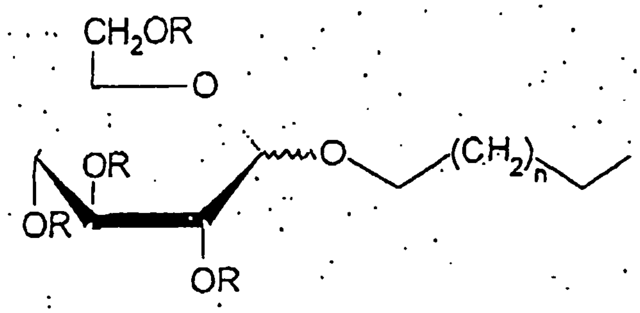

wobei

R = H oder Glucosyl.
n = 5-13,

besonders bevorzugt Caprylyl/Capryl Glucoside, Cetearyl Glucoside, Decylglucosid, Laurylglucosid eingesetzt werden.

**13.** Silikon-in-Wasser-Emulsion nach einem der vorangehenden Ansprüche **dadurch gekennzeichnet, daß** als Aminosäurederivate N-Cocoyl-L-glutaminsäure, Dinatrium L-N-Cocoylglutamat, Dinatrium Lauroylglutamat, Dinatrium N-(1-Oxooctadecyl)-L-Glutamat, Kalium N-Cocoacyl-L-glutamat, Kalium N-Lauroyl-L-glutamate, Triethanolamin N-Cocoyl-L-glutamat, Natrium N-(1-Oxooctadecyl)-L-Glutamat, DL-Pyrrolidonecarbonsäuresalz des L-Cocoylargininethylesters, Triethanolamin N-Cocoyl-L-alaninat, Natrium N-Kokosacylglycinat, N-(Carboxymethyl)-N,N-Bis (2-Hydroxyethyl)-1-Octadecanaminiumhydroxide N-Cocoyl-L-glutamat, besonders bevorzugt Natrium N-CocoylL-glutamat verwendet werden.

**14.** Silikon-in-Wasser-Emulsion nach einem der vorangehenden Ansprüche **dadurch gekennzeichnet, daß** als quaternäre Ammoniumverbindungen Coco Alkyl Ethyldimethylammonium Ethyl Sulfate, [2-[[2-[(2-Carboxyethyl)(2-Hydroxyethyl)Amino]Ethyl]Amino]-2-Oxoet, Cocoyltrimethylammoniumchlorid, Di-C12-15-Alkyldimethylammoniumchlorid, Quaternium-1B, Quaternium-26, Talgtrimethylammoniumchlorid verwendet werden.

**15.** Silikon-in-Wasser-Emulsion nach einem der vorangehenden Ansprüche erhältlich durch Hochdruckhomogenisierung, wobei die Emulsion einer Hochdruckhomogenisierung bei 30 bis 1500 bar, besonders bevorzugt bei 750 bar unterworfen wird.

**16.** Silikon-in-Wasser-Emulsion nach einem der vorangehenden Ansprüche erhältlich durch Anwendung der PIS-Technik **gekennzeichnet durch**

(a) Herstellung einer tensidfreien Wasser-in Silikon-Emulsion bei Temperaturen oberhalb 60°C, besonders bevorzugt oberhalb 50°C,
wobei die tensidfreie Wasser-in Silikon-Emulsion

(a1) mehr als 50 Gew.% Silikonölen in der Fettphase,
(a2) polyethermodifizierte Polysiloxane,
(a3) mindestens einen nichtionischen Emulgator umfasst,

(b) Temperieren auf Temperaturen unterhalb 60°C, besonders bevorzugt unterhalb 50°C,

(c) Zugabe von weniger als 5 Gew.%, besonders bevorzugt weniger als 2 Gew.% Tensiden gewählt aus der Gruppe der tensidischen Betaine, Alkylpolyglycoside, Aminosäurederivate oder quaternäre Ammoniumverbindungen, um eine Phasenumkehr zu einer Silikon-in Wasser-Emulsion zu erreichen.

**17.** Silikon-in-Wasser-Emulsion nach einem der vorangehenden Ansprüche **dadurch gekennzeichnet, daß** das Zahlenmittel der Tröpfchengröße weniger als 1 μm, besonders bevorzugt 50 bis 300 nm, ganz besonders bevorzugt 70 bis 100 nm beträgt.

## Claims

**1.** Cosmetic and/or dermatological silicone-in-water emulsion with a content of silicone oils in the fatty phase of more than 50% by weight, which comprises polyether-modified polysiloxanes, **characterized in that**, as polyether-modified polysiloxane, bis-PEG/PPG-16/16 PEG/PPG 16/16 dimethicone (I) is present.

**2.** Silicone-in-water emulsion according to claim one, **characterized in that** the content of polyether-modified polysiloxanes is 0.25 to 15% by weight, based on the total weight of the emulsion.

**3.** Silicone-in-water emulsion according to one of the preceding claims, **characterized in that** it has a content of at least one nonionic emulsifier.

**4.** Silicone-in-water emulsion according to one of the preceding claims, **characterized in that** the nonionic emulsifiers are chosen from the group of glyceryl stearates, polyethylene glycols and/or alkyl polyglycosides.

**5.** Silicone-in-water emulsion according to one of the preceding claims, **characterized in that** the nonionic emulsifiers are chosen from the group of glyceryl stearate citrate, polyethylene glycol 40, polyethylene glycol 100, polyglycerol-3 methylglucose distearate, glyceryl stearate.

**6.** Silicone-in-water emulsion according to one of the preceding claims, **characterized in that** it has a content of at least one anionic emulsifier.

**7.** Silicone-in-water emulsion according to one of the preceding claims, **characterized in that** the anionic emulsifiers are chosen from the group trilaureth-4 phosphate, cetyl phosphate, sodium cetearyl sulphate, salts of stearic acid.

**8.** Silicone-in-water emulsion according to one of the preceding claims, **characterized in that** the content of nonionic and/or anionic emulsifiers is 0.01 to 5% by weight, based on the total weight of the emulsion.

**9.** Silicone-in-water emulsion according to one of the preceding claims, **characterized in that** the weight ratio of nonionic and/or anionic emulsifiers to polyether-modified polysiloxanes is 0.001 to 20.

**10.** Silicone-in-water emulsion according to one of the preceding claims, **characterized in that** it has a content of surfactants chosen from the group of surface-active betaines, alkyl polyglycosides, amino acid derivatives or quaternary ammonium compounds.

**11.** Silicone-in-water emulsion according to one of the preceding claims, **characterized in that** the surface-active betaines used are alkylpolybetaines, coconut fatty acid amidopropylbetaine, wheatgerm fatty acid amidopropylbetaine, coconut fatty acid and oleic acid amidopropylbetaine, dimethicone propyl PG-betaine, isostearamidopropylbetaine, palmitamidopropyl-betaine, stearamidopropylbetaine, sulphobetaines of the formula $H_{25\text{-}37}C_{12\text{-}18}N^+(CH_3)_2\text{-}(CH_2)_3\text{-}SO_3$, capryl/capramidopropylbetaine, particularly preferably capryl/capramidopropylbetaine.

**12.** Silicone-in-water emulsion according to one of the preceding claims, **characterized in that** the alkyl polyglycosides used are compounds of the formula

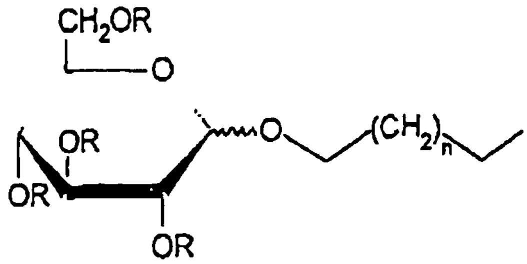

where

R = H or glucosyl,
n = 5-13,

particularly preferably caprylyl/capryl glucosides, cetearyl glucosides, decyl glucoside, lauryl glucoside.

**13.** Silicone-in-water emulsion according to one of the preceding claims, **characterized in that** the amino acid derivatives used are N-cocoyl-L-glutamic acid, disodium L-N-cocoylglutamate, disodium lauroylglutamate, disodium N-(1-oxooctadecyl)-L-glutamate, potassium N-cocoacyl-L-glutamate, potassium N-lauroyl-L-glutamate, triethanolamine N-cocoyl-L-glutamate, sodium N-(1-oxooctadecyl)-L-glutamate, DL-pyrrolidonecarboxylic acid salt of the L-cocoylarginine ethyl ester, triethanolamine N-cocoyl-L-alaninate, sodium N-cocoacylglycinate, N-(carboxymethyl)-N,N-bis(2-hydroxyethyl)-1-octadecanaminium hydroxides, N-cocoyl-L-glutamate, particularly preferably sodium N-cocoyl-L-glutamate.

**14.** Silicone-in-water emulsion according to one of the preceding claims, **characterized in that** the quaternary ammonium compounds used are cocoalkylethyldimethylammonium ethyl sulphates, [2-[[2-[(2-carboxyethyl)(2-hydroxyethyl)amino]-ethyl]amino]-2-oxoet, cocoyltrimethylammonium chloride, di-C12-15-alkyldimethylammonium chloride, quaternium-18, quaternium-26, tallow-trimethylammonium chloride.

**15.** Silicone-in-water emulsion according to one of the preceding claims obtainable by high-pressure homogenization, where the emulsion is subjected to a high-pressure homogenization at 30 to 1500 bar, particularly preferably at 750 bar.

**16.** Silicone-in-water emulsion according to one of the preceding claims obtainable using the PIS technique **characterized by**

(a) preparation of a surfactant-free water-in-silicone emulsion at temperatures above 60°C, particularly preferably above 50°C,
where the surfactant-free water-in-silicone emulsion comprises

(a1) more than 50% by weight of silicone oils in the fatty phase,
(a2) polyether-modified polysiloxanes,
(a3) at least one nonionic emulsifier,

(b) heating to temperatures below 60°C, particularly preferably below 50°C,
(c) addition of less than 5% by weight, particularly preferably less than 2% by weight, of surfactants chosen from the group of surface-active betaines, alkyl polyglycosides, amino acid derivatives or quaternary ammonium compounds in order to achieve phase reversal to a silicone-in-water emulsion.

**17.** Silicone-in-water emulsion according to one of the preceding claims, **characterized in that** the number-average of the droplet size is less than 1 µm, particularly preferably 50 to 300 nm, very particularly preferably 70 to 100 nm.

## Revendications

**1.** Emulsion cosmétique et/ou dermatologique silicone-dans-l'eau, ayant une teneur de la phase grasse en huiles de

silicone supérieure à 50 % en poids, qui contient des polysiloxanes modifiés par un éther, **caractérisée en ce qu'**elle contient, en tant que polysiloxanes modifiés par un éther, du bis-PEG/PPG-16/16 PEG/PPG16/16 Dimethicone.

**2.** Emulsion silicone-dans-l'huile selon l'une des revendication 1, **caractérisée en ce que** la teneur en les polysiloxanes modifiés par un éther est de 0,25 à 15 % en poids par rapport au poids total de l'émulsion.

**3.** Emulsion silicone-dans-l'eau selon l'une des revendications précédentes, **caractérisée en ce qu'**elle contient au moins un émulsifiant non ionique.

**4.** Emulsion silicone-dans-l'eau selon l'une des revendications précédentes, **caractérisée en ce que** les émulsifiants non ioniques sont choisis dans le groupe des stéarates de glycéryle, des polyéthylèneglycols et/ou des alkylpolyglucosides.

**5.** Emulsion silicone-dans-l'eau selon l'une des revendications précédentes, **caractérisée en ce que** les émulsifiants non ioniques sont choisis dans le groupe du stéarate et citrate de glycéryle, du polyéthylèneglycol(40), du polyéthylèneglycol(100), du distéarate de polyglycérol-3-méthylglucose, du stéarate de glycéryle.

**6.** Emulsion silicone-dans-l'eau selon l'une des revendications précédentes, **caractérisée en ce qu'**elle contient au moins un émulsifiant anionique.

**7.** Emulsion silicone-dans-l'eau selon l'une des revendications précédentes, **caractérisée en ce que** les émulsifiants anioniques sont choisis dans le groupe du phosphate de trilaureth-4, du phosphate de cétyle, du cétéarylsulfate de sodium, des sels de l'acide stéarique.

**8.** Emulsion silicone-dans-l'eau selon l'une des revendications précédentes, **caractérisée en ce que** la teneur en les émulsifiants non ioniques et/ou anioniques est de 0,01 à 5 % en poids par rapport au poids total de l'émulsion.

**9.** Emulsion silicone-dans-l'eau selon l'une des revendications précédentes, **caractérisée en ce que** le rapport pondéral des émulsifiants non ioniques et/ou anioniques aux polysiloxanes modifiés par un polyéther est de 0,001 à 20.

**10.** Emulsion silicone-dans-l'eau selon l'une des revendications précédentes, **caractérisée en ce qu'**elle contient des tensioactifs choisis dans l'ensemble comprenant les bétaïnes, les alkylpolyglycosides, les dérivés d'acides aminés ou les dérivés de l'ammonium quaternaire, à effet tensioactif.

**11.** Emulsion silicone-dans-l'eau selon l'une des revendications précédentes, **caractérisée en ce qu'**on utilise en tant que bétaïnes tensioactives des alkylpolybétaïnes, l'amidopropylbétaïne de l'acide gras dérivé de l'huile de coprah, l'amidopropylbétaïne de l'acide gras dérivé du germe de blé, les amidopropyl-bétaïnes de l'acide gras dérivé de l'huile de coprah et de l'acide oléique, la Dimethicone-Propyl-PG-bétaïne, l'isostéaramidopropyl-bétaïne, les palmitamidopropyl-bétaïnes, les stéaramidopropylbétaïnes, les sulfobétaïnes de formule $H_{25-37}C_{12-18}N^{+}(CH_3)_2$-$(CH_2)_3$-$SO_3$, la capryl/ capramidopropyl-bétaïne, d'une manière particulièrement préférée la capryl/capramidopropyl-bétaïne.

**12.** Emulsion silicone-dans-l'eau selon l'une des revendications précédentes, **caractérisée en ce qu'**on utilise en tant qu'alkylpolyglycosides des composés de formule

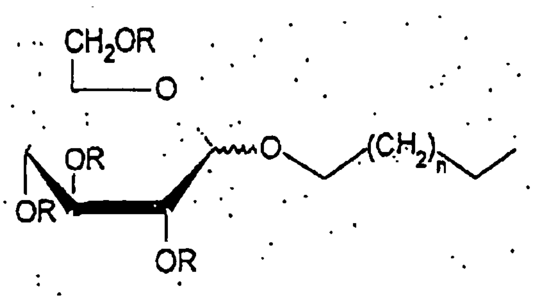

dans laquelle R est H ou le groupe glucosyle, et n vaut 5 à 13, d'une manière particulièrement préférée les caprylyl/caprylglucosides, les cétéarylglucosides, le décylglucoside, le laurylglucoside.

**13.** Emulsion silicone-dans-l'eau selon l'une des revendications précédentes, **caractérisée en ce qu'**on utilise en tant que dérivés d'acides aminés l'acide N-cocoyle-L-glutamique, le L-N-cocoylglutamate disodique, le lauroylglutamate disodique, le N-(1-oxooctadécyl)-L-glutamate disodique, le N-cocoacyl-L-glutamate de potassium, le N-lauroyl-L-glutamate de potassium, le N-cocoyl-L-glutamate de triéthanolamine, le N-(1-oxooctadécyl)-L-glutamate de sodium, le sel de l'acide DL-pyrrolidonecarboxylique de l'ester éthylique de la L-cocoylarginine, le sel de triéthanolamine de la N-cocoyl-L-alanine, le N-coco-acylglycinate de sodium, l'hydroxyde de N-(carboxyméthyl)-N,N-bis(2-hydroxyéthyl)-1-octadécane-aminium, les N-cocoyl-L-glutamates, d'une manière particulièrement préférée le N-cocoyl-L-glutamate de sodium.

**14.** Emulsion silicone-dans-l'eau selon l'une des revendications précédentes, **caractérisée en ce qu'**on utilise en tant que composé de l'ammonium quaternaire l'éthylsulfate de cocoalkyléthyldiméthylammonium, le [2-[[-2-[(2-carboxyéthyl)(2-hydroxyéthyl)amino]éthyl]-amino]-2-oxoet, le chlorure de cocoyltriméthylammonium, le chlorure de di-(alkyle en $C_{12-15}$)-diméthylammonium, le quaternium-18, le quaternium-26, le chlorure de suif-triméthylammonium.

**15.** Emulsion silicone-dans-l'eau selon l'une des revendications précédentes, pouvant être obtenue par homogénéisation sous haute pression, l'émulsion étant soumise à une homogénéisation sous une haute pression de 30 à 1 500 bar et d'une manière particulièrement préférée de 750 bar.

**16.** Emulsion silicone-dans-l'eau selon l'une des revendications précédentes, pouvant être obtenue par utilisation de la technique PIS, **caractérisée par**

(a) préparation d'une émulsion eau-dans-silicone sans tensioactifs, à des températures supérieures à 60°C, d'une manière particulièrement préférée supérieure à 50°C,
l'émulsion eau-dans-silicone sans tensioactifs comprenant

(a1) plus de 50 % en poids d'huiles de silicone dans la phase grasse,
(a2) des polysiloxanes modifiés par un polyéther,
(a3) au moins un émulsifiant non ionique,

(b) équilibrage à des températures inférieures à 60°C, d'une manière particulièrement préférée inférieures à 50°C,
(c) addition de moins de 5 % en poids, d'une manière particulièrement préférée inférieure à 2 % en poids, de tensioactifs choisis dans l'ensemble consistant en les bétaïnes, les alkylpolyglycosides, les dérivés d'acides aminés ou les composés de l'ammonium quaternaire, à effet tensioactif, pour arriver à une inversion de phase conduisant à une émulsion silicone-dans-l'eau.

**17.** Emulsion silicone-dans-l'eau selon l'une des revendications précédentes, **caractérisée en ce que** la taille moyenne en nombre des gouttelettes est inférieure à 1 µm, d'une manière particulièrement préférée de 50 à 300 nm et d'une manière tout particulièrement préférée de 70 à 100 nm.